# EUROPEAN PATENT APPLICATION

(11) **EP 4 328 316 A1**
(43) Date of publication of application: **28.02.2024**
(21) Application number: 21940283.1
(22) Date of filing: 31.05.2021
(51) Int. Cl.: C12P 21/02, C12P 21/06

(54) **PREPARATION METHOD FOR POLYPEPTIDE**

(30) Priority: 21.05.2021 CN 202110554938
(71) Applicant: Asymchem Laboratories (Tianjin) Co., Ltd, Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); JIAO, Xuecheng, Tianjin 300457 (CN); ZHAO, Junqi, Tianjin 300457 (CN); WANG, Lei, Tianjin 300457 (CN); MENG, Xiangyu, Tianjin 300457 (CN); ZHANG, Mujiao, Tianjin 300457 (CN)
(74) Representative: Studio Torta S.p.A.
(86) International application number: PCT/CN2021/097429
(87) International publication number: WO 2022/241831

(57) **Abstract**

Provided is a preparation method for a polypeptide. The preparation method includes the following steps: constructing an engineering strain for fusion-expressing a polypeptide gene with a Sumo tag, and inducing the engineering strain for the soluble expression of a polypeptide; obtaining a fusion protein containing a polypeptide precursor from the engineering strain by purification; cleaving the fusion protein containing the polypeptide precursor by a Ulp1 protease to remove the Sumo tag; and purifying a cleavage product of the Ulp1 protease by a method of acetonitrile combined with heating precipitation or a method of precipitation with hexafluoroisopropanol to obtain the polypeptide. By establishing an efficient technology based on soluble recombinant expression of a medicinal polypeptide or a precursor thereof and establishing a simple purification process based on one-step precipitation purification, further combined with HPLC refinement, a polypeptide purity of over 97% can be achieved.

## Description

### Technical Field

The present invention relates to the technical field of biological medicine, in particular, to a preparation method for a polypeptide.

### Background

Polypeptide drugs have attracted much attention because of their dual properties similar to protein and small molecule drugs. In 2015 alone, the sales of polypeptide drugs accounted for 5% of the drug market, reaching 50 billion dollars, and rising at an annual rate of 9-10%. Therefore, it is urgent to establish efficient and low-cost polypeptide synthesis and purification technologies. Polypeptide synthesis mainly includes a chemical method, a chemical/enzymatic method and a biological method. The chemical method requires complex protection and deprotection processes, require a large amount of toxic reagents, and may produce racemates; the chemical/biological method usually uses a chemical method to synthesize polypeptide, and further connect multiple peptide segments through a specific peptide ligase, the process is relatively complex, and the connection efficiency and specificity are affected by a polypeptide sequence; the biological method includes hydrolysis of plant protein or animal protein by a hydrolase and extraction to obtain a specific functional polypeptide, but usually the production is low, the cycle is long, the pollution is serious, and it is difficult to achieve industrial production; another common method is recombinant expression, that is to introduce a target gene expression element into prokaryotes or eukaryotes by a technical means of genetic engineering, and achieve efficient synthesis of a target polypeptide through microbial fermentation. However, because the polypeptide itself is relatively short, it is difficult to form a specific spatial structure, which makes it very easy to be degraded by a protease of an expression host. In addition, the complex purification process reduces the product yield, and increases costs.

At present, polypeptides produced by the recombinant method include polypeptide antibiotics, interferons, GLP-1 analog, insulin, etc. Liraglutide is a polypeptide drug developed by Novo Nordisk for the treatment of type II diabetes, with annual sales of billions of dollars, its original synthesis method is based on an engineering strain secreted and expressed by *Saccharomyces cerevisiae,* however, due to the role of its extracellular protease, the product is easy to be degraded, the yield is low, and the highest expression level after knocking out the protease is only 59 mg/L. Liraglutide is fused and expressed in Pichia pastoris, and the expression level after 5 days of fermentation is only 26 mg/L. As a commonly used host, *Escherichia coli* has been widely used for polypeptide biosynthesis, but there is also a risk of protease degradation, which can be addressed through fusion expression. At present, Liraglutide is mainly fused and expressed as inclusion bodies, such as a KSI tag, intein and enterokinase cleavage site-Liraglutide, etc. Although the expression quantity of the inclusion body is high and protease degradation can be avoided, the complex denaturation and refolding process requires a large number of detergents, such as urea or guanidinium chloride. The complex purification process leads to very low final yield. Meanwhile, in order to completely remove the fusion tag, enterokinase is usually required, which is not only difficult to express but also causes non-specific cleavage. Recently, the use of an inclusion body constructed with an MFH fusion tag for expression in combination with formic acid cleavage and a purification process under a denaturation condition avoids the refolding process, but needs to use urea. Further, Liraglutide containing a Gly at an N-terminal is obtained through Tev protease cleavage, and Tev enzyme has low biological activity and produces non-specific cleavage, which leads to cumbersome production steps and high cost. Other fusion tags such as GST, MBP, TrxA, etc. are often used as soluble expression tags, and some peptides have obtained soluble expression through these tags. However, the molecular weight of these tags is relatively large compared to some target peptides, resulting in a relatively low yield of target peptides after removing the tags. In addition, Liraglutide and derivatives thereof are mostly in a polymer state, and purification needs to be carried out under a denaturation condition. Current purification steps are complex and the yield is low.

Overall, the prior art mainly has the following problems: 1) chemical synthesis: the process is complex, chemical reagents are toxic, it is possible to generate a racemate, the cycle is long, and the quality control of product is relatively difficult; 2) expression with inclusion body: the denaturation, refolding and purification processes are complex, a large amount of urea is used, and the enterokinase or Tev used in the cleavage process is expensive, and is prone to produce non-specific cleavage; the complex denaturation and refolding process leads to a very complex purification process and a decrease in yield; 3) soluble expression: soluble expression tags currently used mainly include TrxA, GST and MBP, target peptide segments and tags need to be added with enzyme cleavage sites, the commonly used enzymes are usually difficult to express and will cause non-specific cleavage, and Sumo tags have not been used for the study of fusion expression of Liraglutide precursor.

### SUMMARY

The present invention aims to provide a preparation method for a polypeptide, so as to solve the technical problems of complex polypeptide purification steps and low yield in the prior art.

In order to achieve the above purpose, according to one aspect of the present invention, there is provided a preparation method for a polypeptide. The preparation method includes the following steps: constructing an engineering strain for fusion-expressing a polypeptide gene with a Sumo tag, and inducing the engineering strain to soluble-express the polypeptide; obtaining a fusion protein containing a polypeptide precursor from the engineering strain by purification; cleaving the fusion protein containing the polypeptide precursor by using a Ulp1 protease to remove the Sumo tag; and purifying a cleavage product of the Ulp1 protease by a method of acetonitrile combined with heating precipitation or a method of precipitation with hexafluoroisopropanol to obtain a polypeptide.

Further, the polypeptide is a Liraglutide precursor, Nesiritide or Teriparatide.

Further, the Ulp1 protease is obtained by constructing a Ulp1 protease expression strain and inducing expression, wherein the Ulp1 protease is co-expressed with a chaperone.

Further, the chaperone is a GroEUS chaperone.

Further, the method of acetonitrile combined with heating precipitation includes: adjusting pH of the cleavage product of the Ulp1 protease to 5.6, then adding acetonitrile, and performing heat treatment for 0.5-3 h at 60-80°C (preferably heat treatment for 2 h at 70°C) after mixing uniformly, and then centrifuging to separate supernatant and precipitate.

Further, the acetonitrile is an aqueous solution of acetonitrile with a mass percentage content of 20-70%.

Further, the step of obtaining the fusion protein containing a polypeptide precursor from the engineering strain by purification includes: obtaining a crude solution after ultrasonic disruption, centrifugation, and filtration with membrane of the engineering strain, and then purifying with affinity chromatography or an anion column to obtain the fusion protein.

Further, the method of precipitation with hexafluoroisopropanol includes: adjusting pH of the cleavage product of the Ulp1 protease to 5.6, then adding hexafluoroisopropanol, after mixing uniformly, precipitating for 1 h at a room temperature, and then conducting centrifugation to separate a supernatant and a precipitate.

Further, the hexafluoroisopropanol is an aqueous solution of hexafluoroisopropanol with a mass percentage content of 20-70%; preferably, the hexafluoroisopropanol is an aqueous solution of hexafluoroisopropanol with a mass percentage content of 50%.

Further, the preparation method includes a step of purifying a target polypeptide by HPLC.

According to the present invention, by establishing an efficient technology based on soluble recombinant expression of a medicinal polypeptide or a precursor thereof and establishing a simple purification process based on one-step precipitation purification, further combined with HPLC refinement, a polypeptide purity of over 97% can be achieved.

### Brief Description of the Drawings

The accompanying drawings of the specification forming a part of the present application are used to provide a further understanding of the present invention. The schematic embodiments and explanations thereof of the present invention are used to explain the present invention and do not constitute an improper limitation of the present invention. In the drawings:
FIG. 1 shows an SDS-PAGE electrophoretogram of a target protein of expression of a Sumo-Lira fusion protein and purified by affinity chromatography in Embodiments 1 and 5, where Lane 1: protein molecular weight standard; Lane 2: Sumo-Lira soluble expression part; Lane 3: flow-through sample; Lane 4: product eluted with 60 mM imidazole; Lane 5: product eluted with 300 mM imidazole;
FIG. 2 shows an SDS-PAGE electrophoretogram of target proteins of Sumo-Nesi and Sumo-Teri fusion expression in Embodiments 2 and 3, where Lane 1: protein molecular weight standard; Lane 2 and 3: Sumo-Teri soluble expression parts; Lane 4: Sumo-Teri precipitate part; Lane 5: Sumo-Nesi soluble expression part; Lane 6: Sumo-Nesi precipitate part;
FIG. 3 shows an SDS-PAGE electrophoretogram of a target protein in a expression optimization process of Ulp1 in Embodiment 4, where Lane 1: induction of Ulp1 by IPTG, 37°C, 6 h, soluble expression part; Lane 2: protein molecular weight standard; Lane 3: induction of Ulp1 by IPTG, 37°C, 6 h, precipitate part; Lane 4: induction of Ulp1 and GroEL/S co-expression strain by IPTG, 37°C, 6 h, soluble expression part; Lane 5: induction of Ulp1 and GroEUS co-expression strain by IPTG, 37°C, 6 h, precipitate part;
FIG. 4 shows an SDS-PAGE electrophoretogram of a Sumo-Lira target protein purified by anion column chromatography in Embodiment 5, where Lane 1: protein molecular weight standard; Lane 2: Sumo-Lira fragmentation liquid supernatant; Lane 3: flow-through sample; Lane 4: elution with 50 mM NaCl; Lane 5: elution with 100 mM NaCl; Lane 6: elution with 500 mM NaCl; Lane 7: elution with 700 mM NaCl;
FIG. 5 shows an SDS-PAGE electrophoretogram of a target protein of Ulp1 purified by affinity chromatography in Embodiment 7, where Lane 1: protein molecular weight standard; Lane 2: Ulp1 soluble expression part; Lane 3: flow-through sample; Lane 4: elution with 500 mM imidazole;

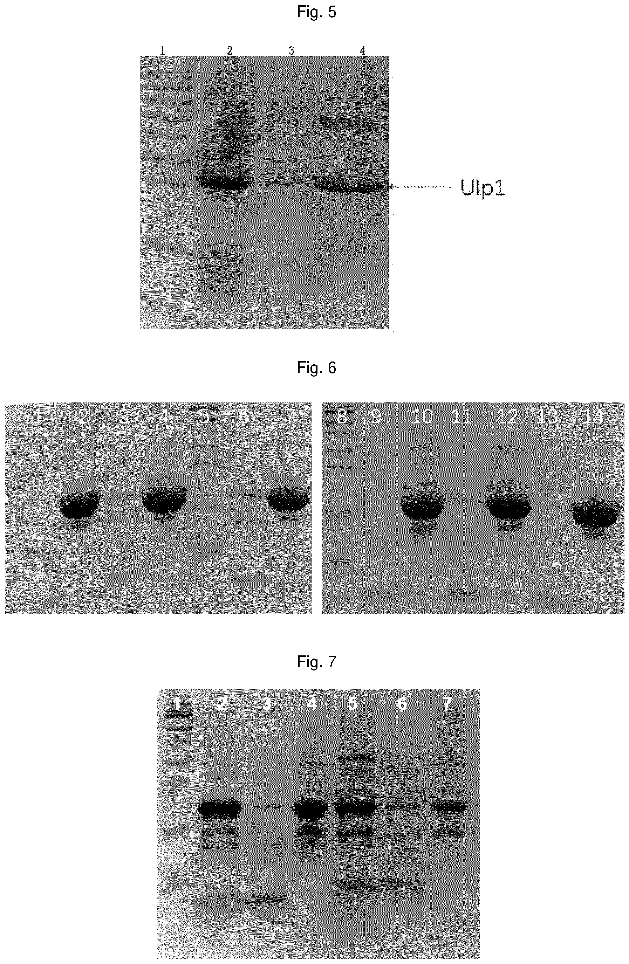

FIG. 6 shows an SDS-PAGE electrophoretogram of a target protein of a Liraglutide precursor precipitated with acetonitrile in Embodiment 9, where Lane 1: 20% acetonitrile, pH 5.6, treatment at 70°C, supernatant part; Lane 2: 20% acetonitrile, pH 5.6, treatment at 70°C, precipitate part; Lane 3: 30% acetonitrile, pH 5.6, treatment at 70°C, supernatant part; Lane 4: 30% acetonitrile, pH 5.6, treatment at 70°C, precipitate part; Lane 5: protein molecular weight standard; Lane 6: 40% acetonitrile, pH 5.6, treatment at 70°C, supernatant part; Lane 7: 40% acetonitrile, pH 5.6, treatment at 70°C, precipitate part; Lane 8: protein molecular weight standard; Lane 9: 50% acetonitrile, pH 5.6, treatment at 70°C, supernatant part; Lane 10: 50% acetonitrile, pH 5.6, treatment at 70°C, precipitate part; Lane 11: 60% acetonitrile, pH 5.6, treatment at 70°C, supernatant part; Lane 12: 60% acetonitrile, pH 5.6, treatment at 70°C, precipitate part; Lane 13: 70% acetonitrile, pH 5.6, treatment at 70°C, supernatant part; Lane 14: 70% acetonitrile, pH 5.6, treatment at 70°C, precipitate part;
FIG. 7 shows an SDS-PAGE electrophoretogram of Nesi and Teri target proteins precipitated with acetonitrile in Embodiment 9, where Lane 1: protein molecular weight standard; Lane 2: Sumo-Nesi cleaved by Ulp1; Lane 3: Ulp1 cleavage system of Sumo-Nesi treated by precipitation method, supernatant part; Lane 4: Ulp1 cleavage system of Sumo-Nesi treated by precipitation method, precipitate part; Lane 5: Sumo-Teri cleaved by Ulp1; Lane 6: Ulp1 cleavage system of Sumo-Teri treated by precipitation method, supernatant part; Lane 7: Ulp1 cleavage system of Sumo-Teri treated by precipitation method, precipitate part;
FIG. 8 shows an SDS-PAGE electrophoretogram of a Liraglutide precursor purified by an HFIP precipitation method in Embodiment 9, where Lane 1: supernatant selectively precipitated with 18% HFIP; Lane 2: precipitate part selectively precipitated with 18% HFIP; Lane 3: supernatant selectively precipitated with 50% HFIP; Lane 4: precipitate part selectively precipitated with 50% HFIP; Lane 5: supernatant selectively precipitated with 70% HFIP; Lane 6: precipitate part selectively precipitated with 70% HFIP;
FIG. 9 shows a chromatogram of preparative HPLC purification of a Liraglutide precursor in Embodiments 9 and 10;
FIG. 10 shows a chromatogram of purity analysis of a Liraglutide precursor in Embodiment 10;
FIG. 11 shows a chromatogram of preparative HPLC purification of Nesi in Embodiment 10;
FIG. 12 shows a chromatogram of purity analysis of Nesi in Embodiment 10;
FIG. 13 shows a chromatogram of preparative HPLC purification of Teri in Embodiment 10;
FIG. 14 shows a chromatogram of purity analysis of Teri in Embodiment 10;
FIG. 15 shows a molecular weight of Liraglutide analyzed by mass spectrometry in Embodiment 11;
FIG. 16 shows a molecular weight of Nesiritide analyzed by mass spectrometry in Embodiment 11; and
FIG. 17 shows a molecular weight of Teriparatide analyzed by mass spectrometry in Embodiment 11.

### Detailed Description of the Embodiments

It should be illustrated that the embodiments in the present application and the features in the embodiments can be combined with each other in the case of no conflict. The present invention will be described in detail below with reference to the drawings and in combination with the embodiments.

### Explanation of nouns

Polypeptide is a peptide composed of 10-50 amino acid residues.

Liraglutide, an analog of human Glucagon-like peptide-1 (GLP-1), is called Lira for short.

Nesiritide is called Nesi for short.

Teriparatide is called Teri for short.

Polypeptide precursor, in the present invention, refers to a polypeptide containing a Sumo tag.

According to an exemplary implementation of the present invention, there is provided a preparation method for a polypeptide. The preparation method includes the following steps: constructing an engineering strain for fusion-expressing a polypeptide gene with a Sumo tag, and inducing the engineering strain to soluble-express the polypeptide; obtaining a fusion protein containing a polypeptide precursor from the engineering strain by purification; cleaving the fusion protein containing the polypeptide precursor by using a Ulp1 protease to remove the Sumo tag; and purifying a cleavage product of the Ulp1 protease by a method of acetonitrile combined with heating precipitation or a method of precipitation with hexafluoroisopropanol to obtain a polypeptide.

According to the present invention, based on a method that the soluble expression of protein can be promoted by a Sumo tag which, as a fusion tag, can be efficiently removed by the Ulp1 protease (SUMO protease) that specifically recognizes a tertiary structure of the Sumo tag, and non-specific cleavage will not be caused, a target polypeptide is obtained, avoiding the problem of inclusion body denaturation, and an engineering strain for Sumo tag fusion expression of a polypeptide gene is constructed.

Exemplarily, the polypeptide in the present invention may be a Liraglutide precursor, Nesiritide or Teriparatide.

In addition, with regard to the problem of the expensive protease required for removing fusion expression tags and having a possibility to cause non-specific cleavage, a strategy of specific cleavage of the Sumo tag by Ulp1 is adopted, Ulp1 recognizes a spatial structure of the Sumo tag and only cleaves at specific site; in order to obtain an efficient Ulp1 engineering strain and facilitate the purification of Ulp1, a strain co-expressing Ulp1 and a chaperone is constructed, preferably, the chaperone is a GroEUS chaperone, greatly improving the expression level of Ulp1.

According to an exemplary implementation of the present invention, the method of acetonitrile combined with heating precipitation includes: adjusting pH of the cleavage product of the Ulp1 protease to 5.6 (obtained by combining data of isoelectric points), then adding acetonitrile, after mixing well, conducting heat treatment for 0.5-3 h at 60-80°C (preferably heat treatment for 2 h at 70°C), and then conducting centrifugation to separate a supernatant and a precipitate. Preferably, the acetonitrile is an aqueous solution of acetonitrile with a mass percentage content of 20-70%, and has a good preliminary purification effect at concentrations ranging from 20% to 70%.

In an exemplary implementation of the present invention, the obtaining a fusion protein containing a polypeptide precursor from the engineering strain by purification includes: obtaining a crude solution after ultrasonic disruption, centrifugation, and filtration with membrane of the engineering strain, and then purifying with affinity chromatography or an anion column to obtain the fusion protein.

According to an exemplary implementation of the present invention, the method of precipitation with hexafluoroisopropanol includes: adjusting pH of the cleavage product of the Ulp1 protease to 5.6, then adding hexafluoroisopropanol, after mixing well, conducting precipitation for 1 h at a room temperature, and then conducting centrifugation to separate a supernatant and a precipitate; preferably, the hexafluoroisopropanol is an aqueous solution of hexafluoroisopropanol with a mass percentage content of 20-70%; preferably, after selective precipitation with 50% hexafluoroisopropanol, the purity of a Liraglutide precursor in the supernatant is about 90%.

Preferably, the preparation method further includes a step of purifying a target polypeptide by HPLC. The Liraglutide precursor is easy to aggregate. The method of treatment with organic solvent and reverse phase purification is adopted in the present invention, which can avoid the purification problem caused by an aggregation effect.

The beneficial effects of the present application will be further illustrated below in combination with the embodiments.

### Embodiment 1

### Construction of gene engineering strain for fusion expression of Liraglutide precursor with Sumo tag (called Lira for short)

A strategy for constructing a polypeptide based on soluble expression was specifically to use a Sumo tag to fuse with a target polypeptide sequence to be constructed on a pET-28a(+) or pET-22b(+) expression vector. A Sumo-Lira sequence after codon optimization was subjected to total gene synthesis by GENEWIZ, introducing an Ndel cleavage site at a 5'-terminal of the sequence, and introducing an Xhol cleavage site at a 3'-terminal of the sequence were conducted for construction on a pUC57 cloning vector, with the following sequence:
SEQ ID NO: 1, CATATGGGCGGCAGTCTGCAAGATAGCGAAGTGAATCAAGAAGCGAAGCCAGAAGTGAAAC CGGAAGTTAAACCGGAGACCCACATCAATCTGAAGGTGAGCGACGGCAGCAGCGAGATCT TCTTCAAGATCAAGAAGACGACCCCGCTGCGTCGTCTGATGGAAGCCTTCGCCAAACGCC AAGGCAAAGAAATGGACAGTCTGCGCTTTCTGTACGATGGTATCCGCATCCAAGCCGATCA AGCCCCGGAAGATCTGGACATGGAGGACAACGACATCATCGAGGCGCATCGCGAACAGAT CGGCGGCCATGCCGAAGGCACCTTCACCAGCGATGTTAGCAGCTATCTGGAAGGCCAAGC CGCCAAAGAATTCATCGCGTGGCTGGTTCGCGGCCGCGGTTAGCTCGAG. Where an underlined part was the Sumo tag, followed by a Liraglutide precursor sequence.

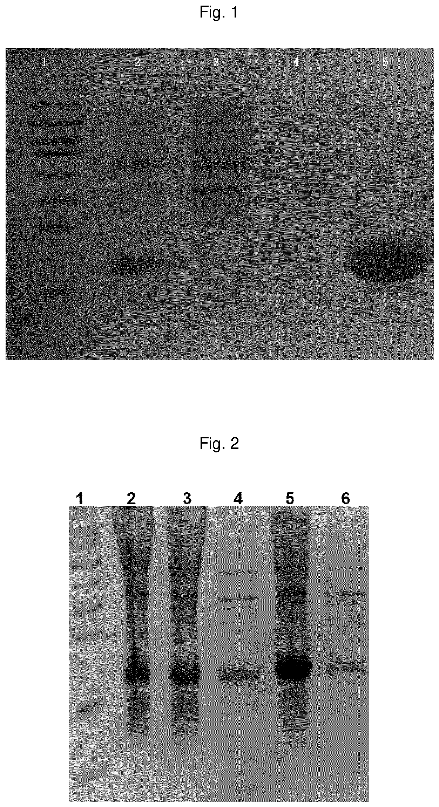

A synthesized gene segment was digested with Ndel and Xhol enzymes from pUC57-Sumo-Lira, and after gel extraction, it was ligated with pET-28a(+) or pET-22b(+) digested with the same enzymes overnight at 16°C, and then transformed into a BL21(DE3) competent cell. Several colonies were subjected to sequencing analysis to obtain a correct clone expression vector pET-28a-Sumo-Lira or pET-22b-Sumo-Lira. 3 correct clones were selected from each transformation and activated as seed cultures for pre-screening with a 250 mL shake flask, and the optimal clone was selected from them as a final expression strain. 4 mL of BL21(DE3) strain containing a recombinant plasmid was taken and inoculated into a 2 L flask containing 600 mL LB medium. When shaking culture was conducted at 37°C, 200 rpm until OD600 was 1.0, IPTG with a final concentration of 1 mM was added for induction at 37°C for 6 h, and after the induction was completed, centrifugation was conducted at 4°C to collect a bacterial cell. Through ultrasonic disruption, a supernatant was collected, and 12% separation gel SDS-PAGE results showed that a target protein was mainly soluble expressed (FIG. 1).

### Embodiment 2

### Construction of gene engineering strain for fusion expression of Nesiritide with Sumo tag (called Nesi for short)

The strategy of Sumo tag fusion expression was also adopted, and the strategy of vector construction was the same as Embodiment 1. A synthesized gene sequence was as follows:
SEQ ID NO: 2, CATATGGGCGGCAGTCTGCAAGATAGCGAAGTGAATCAAGAAGCGAAGCCAGAAGTGAAAC CGGAAGTTAAACCGGAGACCCACATCAATCTGAAGGTGAGCGACGGCAGCAGCGAGATCT TCTTCAAGATCAAGAAGACGACCCCGCTGCGTCGTCTGATGGAAGCCTTCGCCAAACGCC AAGGCAAAGAAATGGACAGTCTGCGCTTTCTGTACGATGGTATCCGCATCCAAGCCGATCA AGCCCCGGAAGATCTGGACATGGAGGACAACGACATCATCGAGGCGCATCGCGAACAGAT CGGCGGCTCTCCGAAAATGGTTCAGGGTTCTGGTTGCTTCGGTCGTAAAATGGACCGTATC TCTTCTTCTTCTGGTCTGGGTTGCAAAGTTCTGCGTCGTCACTAGCTCGAG. Where an underlined part was the Sumo tag, followed by a Nesiritide sequence.

A gene was digested with enzymes and ligation to be constructed on pET-28a(+). Sequencing was conducted to obtain a correct expression plasmid pET-28a-Sumo-Nesi and a recombinant BL21(DE3) strain containing the expression plasmid. By using the same method as Sumo-Lira, after induction with IPTG, a fusion protein was soluble expressed (FIG. 2).

### Embodiment 3

### Construction of gene engineering strain for fusion expression of Teriparatide with Sumo tag (called Teri for short)

The strategy of Sumo tag fusion expression was also adopted, and the strategy of vector construction was the same as Embodiment 1. A synthesized gene sequence was as follows: 
SEQ ID NO: 3,
CATATGGGCGGCAGTCTGCAAGATAGCGAAGTGAATCAAGAAGCGAAGCCAGAAGTGAAAC
 
CGGAAGTTAAACCGGAGACCCACATCAATCTGAAGGTGAGCGACGGCAGCAGCGAGATCT
TCTTCAAGATCAAGAAGACGACCCCGCTGCGTCGTCTGATGGAAGCCTTCGCCAAACGCC
AAGGCAAAGAAATGGACAGTCTGCGCTTTCTGTACGATGGTATCCGCATCCAAGCCGATCA
AGCCCCGGAAGATCTGGACATGGAGGACAACGACATCATCGAGGCGCATCGCGAACAGAT
CGGCGGCTCTGTTTCTGAAATCCAGCTGATGCACAACCTGGGTAAACACCTGAACTCTATG
GAACGTGTTGAATGGCTGCGTAAAAAACTGCAGGACGTTCACAACTTCTAACTCGAG.
 Where an underlined part was the Sumo tag, followed by a Teriparatide sequence.

A gene was digested with Ndel and Xhol enzymes and ligation to be constructed on pET-28a(+). Sequencing was conducted to obtain a correct expression plasmid pET-28a-Sumo-Teri and a recombinant BL21 (DE3) strain containing the expression plasmid. By using the same expression condition as Sumo-Lira, after induction with IPTG, a fusion protein was soluble expressed (FIG. 2).

### Embodiment 4

### Construction of strain for Ulp1 protease expression

A C-terminal part (D390 to K621) of a Ulp1 protease was directly amplified from *Saccharomyces cerevisiae* S288C genome by a PCR method, and primers used were Ulp1-F (SEQ ID NO: 4): gggcatatgGATCTTAAAAAAAAGAAAGAACAATTGGCCAAGAAGAAACTTG and Ulp1-R (SEQ ID NO: 5): Gggctcgaggtattttaaagcgtcggttaaaatcaaatgggc. A gene sequence was as follows (alternatively, artificial synthesis could be carried out in the following sequence):

SEQ ID NO : :6 ,
GATCTTAAAAAAAAGAAAGAACAATTGGCCAAGAAGAAACTTGTTCCTGAATTAAATGAAAAA
GACGATGACCAAGTACAAAAAGCTTTGGCATCTAGAGAAAATACTCAGTTAATGAATAGAGAT
AATATAGAGATAACAGTACGTGATTTTAAGACCTTGGCACCACGAAGATGGCTAAATGACACT
ATCATTGAGTTTTTTATGAAATACATTGAAAAATCTACCCCTAATACAGTGGCGTTTAATTCATT
TTTCTATACCAATTTATCAGAAAGGGGTTATCAAGGCGTCCGGAGGTGGATGAAGAGAAAGA
AGACACAAATTGATAAACTTGATAAAATCTTTACACCAATAAATTTGAACCAATCCCACTGGG
CGTTGGGCATAATTGATTTAAAAAAGAAAACTATAGGTTACGTAGATTCATTATCGAATGGTCC
AAATGCTATGAGTTTCGCTATACTGACTGACTTGCAAAAATATGTTATGGAGGAAAGTAAGCA
TACAATAGGAGAAGACTTTGATTTGATTCATTTAGATTGTCCGCAGCAACCAAATGGCTACGA
CTGTGGAATATATGTTTGTATGAATACTCTCTATGGAAGTGCAGATGCGCCATTGGATTTTGAT
TATAAAGATGCGATTAGGATGAGAAGATTTATTGCCCATTTGATTTTAACCGACGCTTTAAAAT
AG.

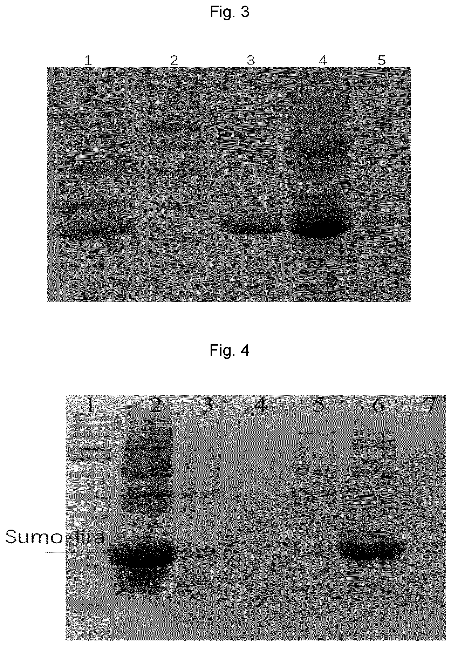

An amplified gene fragment was digested with Ndel and Xhol enzymes and then ligated to pET-28a(+) to obtain an expression vector pET-28a-Ulp1, which was transformed into BL21(DE3), and transformants were sequenced. 3 Clones with correct sequencing were selected as seed culturesfor pre-screening with a 250 mL shake flask, and the optimal clone was selected from them as a final expression strain. 4 mL of BL21(DE3) strain containing a recombinant plasmid was taken and inoculated into a 2 L flask containing 600 mL of an LB medium. When shaking culture was conducted at 37°C, 200 rpm until OD600 was 1.0, IPTG with a final concentration of 1 mM was added for induction at 37°C for 6 h, and after the induction was completed, centrifugation was conducted at 4°C to collect a bacterial cell. Through ultrasonic disruption, a supernatant was collected, and 12% separation gel SDS-PAGE results showed that a target protein was in part soluble expressed. In order to further improve the expression level of Ulp1, the expression vector pET-28a-Ulp1 and an expression plasmid pGRO7 of a chaperone GroEUS were co-expressed. SDS-PAGE results showed that the expression level of Ulp1 was further improved (FIG. 3).

### Embodiment 5

### Purification of Sumo-Lira

An expressed Sumo-Lira bacterial sludge was re-suspended at a bacterial concentration of 20%, and subjected to ultrasonic disruption (5 s ultrasound, 6 s interval, 35% power), centrifugation, and 0.45 µm filtration membrane to obtain a crude solution, which was then purified by affinity chromatography (an AKTA system assembled with 5 mL HisTrap HP). The specific process was as follows: a sample after filtration with filtration membrane was loaded to the purification column at a flow rate of 5 mL/min, and then washed with a binding buffer (20 mM Tris-HCl, 500 mM NaCl, pH 7.4) until an unbound protein was completely eluted, then, 4 column volumes of elution buffer with 60 mM imidazole was used to eluted the impurity protein, and a target protein was then eluted at 500 mM imidazole (FIG. 1). The sample could be directly digested with Ulp1 enzyme without desalination and removal of imidazole. 35-40 mg of a purified Sumo-Lira fusion protein could be obtained from 1 g of the bacterial sludge, 15.2 mg of purified Sumo-Nesi could be obtained from 1 g of the bacterial sludge, and 18.3 mg of purified Sumo-Teri could be obtained from 1 g of the bacterial sludge (which could be further improved after condition optimization).

In order to reduce costs, the expressed Sumo-Lira was attempted to be purified by an anionic column. A fusion protein was obtained using the same method as affinity chromatography. An AKTA system used was assembled with an anionic column (Q FF, 5 mL), a flow rate of 5 mL/min and a binding buffer with 50 mM Tris-HCl and pH 8.0. After being loaded, the sample was washed with the binding buffer until an unbound protein was completely eluted, and then gradiently eluted with 50 mM Tris-HCl, pH 8.0 and 1 M NaCl to obtain a target protein at a 500 mM gradient. SDS-PAGE analysis showed that the target protein could reach a purity of over 80% (FIG. 4). Through test, the purified sample in this step could also be directly cleaved by Ulp1 without the need for desalination.

### Embodiment 6

### Purification of Sumo-Teri and Sumo-Nesi

A fusion protein was obtained from a bacterial sludge obtained by induction expression of the recombinant strain by the same method as the Sumo-Lira sample, and further purified likewise by the same method of affinity chromatography to obtain a target fusion protein.

### Embodiment 7

### Purification of Ulp1

An expressed Ulp1 bacterial sludge was re-suspended at a bacterial concentration of 10%, subjected to ultrasonic disruption (5 s ultrasound, 6 s interval, 35% power), centrifugation, and 0.45 µm filtration membrane, and then purified by affinity chromatography (an AKTA system assembled with 5 mL HisTrap HP). The specific process was as follows: a sample after filtration with filtration membrane was loaded at a flow rate of 4 mL/min, and then washed with a binding buffer (20 mM Tris-HCl, 500 mM NaCl, pH 7.4) until an unbound protein was completely eluted, then, 4 column volumes of impure protein were eluted with 60 mM imidazole, and a target protein was then eluted at 500 mM. Results were shown by SDS-PAGE in FIG. 5, a purity of a target protein reached about 70%, and the obtained target protein could be directly used for cleavage of fusion proteins Sumo-Lira, Sumo-Teri and Sumo-Nesi (products in Embodiments 5 and 6) without desalination.

### Embodiment 8

### Cleavage

Ulp1 cleavage reaction was carried out at 30°C, with a specific process as follows: a mass ratio of Sumo-Lira (product in Embodiment 5) purified with 50 mM Tris-HCl, 10 mM DTT and pH 8.0 to Ulp1 was 10:1 to 1:1 (mg/mg), samples were taken at different times, the cleavage efficiency was detected by Tricine-SDS-PAGE, and whether a target polypeptide was generated was detected. The cleavage efficiency for 24 hours could reach over 80%. The cleavage conditions of Sumo-Teri and Sumo-Nesi were the same as those of Sumo-Lira.

### Embodiment 9

### Purification of target polypeptide by acetonitrile/heating precipitation method

After cleavage in Embodiment 8 was completed, pH was adjusted to 5.6, acetonitrile of different final concentrations (20%, 30%, 40%, 50%, 60%, 70%) were then added into a reaction system, after mixing well, heat treatment was conducted at 70°C for 2 h, and then centrifugation was conducted at 12000 rpm to separate a supernatant and a precipitate. The situation of distribution of a polypeptide was detected by Tricine-SDS-PAGE. Results showed that under the combined conditions, acetonitrile had a good preliminary purification effect at concentrations ranging from 20% to 70%, and after treatment, the purity of a crude Liraglutide precursor could reach over 90% (FIG. 6), the purity of Nesi could reach 80%, and the purity of Teri could reach 50% (FIG. 7). The acetonitrile in a crude polypeptide was further removed by rotary evaporation or freeze-drying, the crude polypeptide was then re-dissolved in 50 mM Tris-HCl with pH 7.0 and preparative HPLC purification was conducted.

### Precipitation of Liraglutide precursor by hexafluoroisopropanol (HFIP)

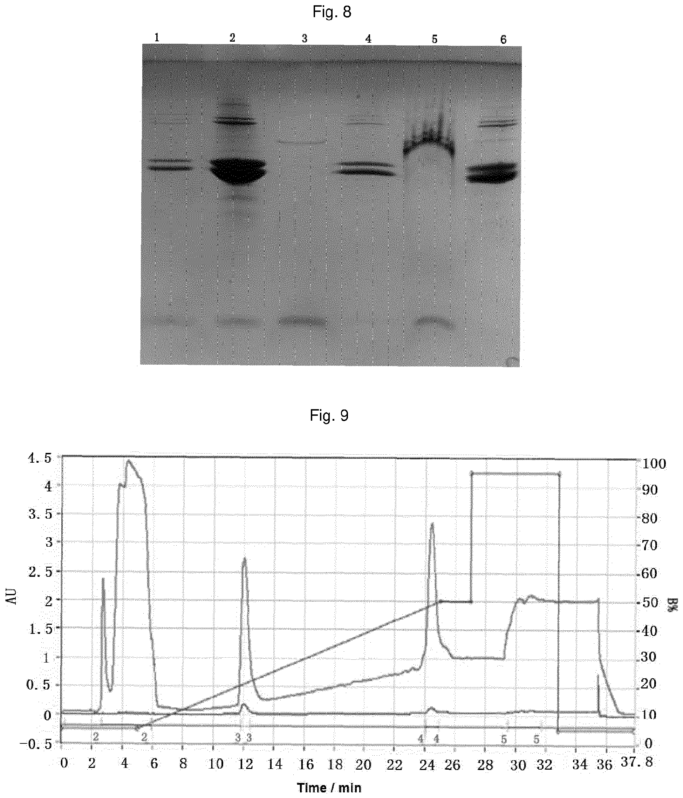

After cleavage in Embodiment 8 was completed, pH was adjusted to 5.6, 10%-70% (v/v) of HFIP was added into a reaction system respectively, precipitation was conducted at a room temperature for 1 h, centrifugation was conducted to collect a supernatant and a precipitate, and the effect of selective precipitation of different concentrations of HFIP on the Liraglutide precursor was detected by Tricine-SDS-PAGE. Results were shown in FIG. 9: after selective precipitation with 50% HFIP, the concentration of the Liraglutide precursor in the supernatant was about 90% (FIG. 8). The HFIP in a crude polypeptide was further removed by rotary evaporation or freeze-drying, the crude polypeptide was then re-dissolved in 50 mM Tris-HCl with pH 7.0 and preparative HPLC purification was conducted.

### Embodiment 10

### Preparative HPLC purification of target polypeptide

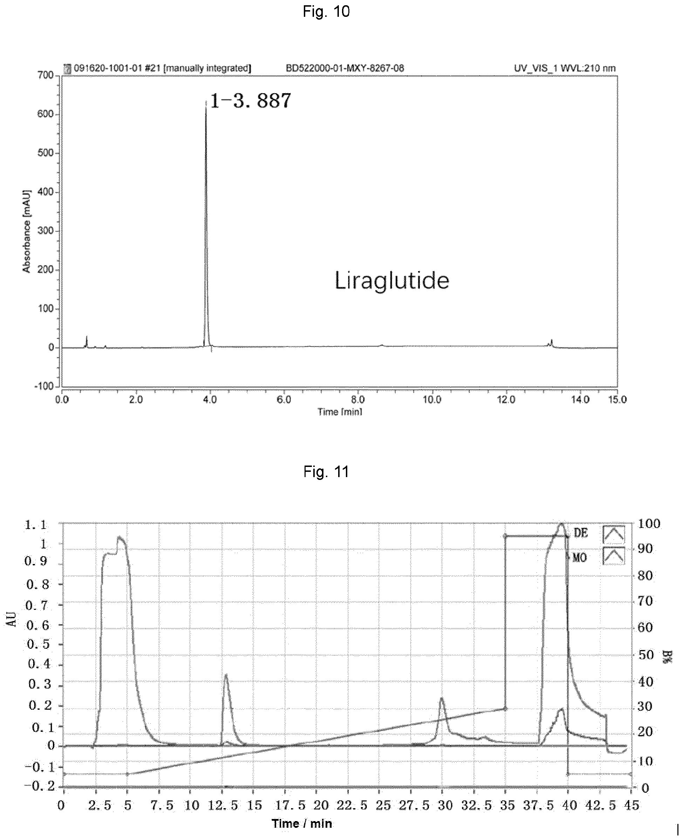

For the product in Embodiment 9, UniSil AQ C18 10 µm 21.5*250 mm was used, and buffers used were buffer A, i.e., 0.1% TFA, and buffer B, i.e., acetonitrile. A gradient elution method used for Lira was as follows: 0 min with 5%B, 5 min with 5%B, 25 min with 50%B, 27 min with 95%B, 33 min with 95%B, 38 min with 5%B, ultraviolet detector of 210 nm, flow rate of 25 mL/min, temperature of 25°C. Results were shown in FIG. 9: Lira was eluted around 24 min. The sample was then collected, subjected to rotary evaporation to remove most of the solvent, and then freeze-dried. The purity was detected by HPLC, and results showed that the purity of Lira reached over 98% (FIG. 10).

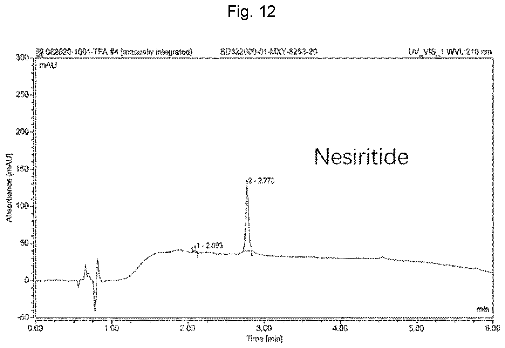

A gradient elution method used for Nesi was as follows: 5 min with 5%B, 35 min with 30%B, 40 min with 95%B, ultraviolet detector of 210 nm, flow rate of 25 mL/min, temperature of 25°C. Results were shown in FIG. 11: Nesi was eluted at 30 min. The sample was then collected, subjected to rotary evaporation to remove most of the solvent, and then freeze-dried. The purity was detected by HPLC, and results showed that the purity of Nesi reached over 98% (FIG. 12).

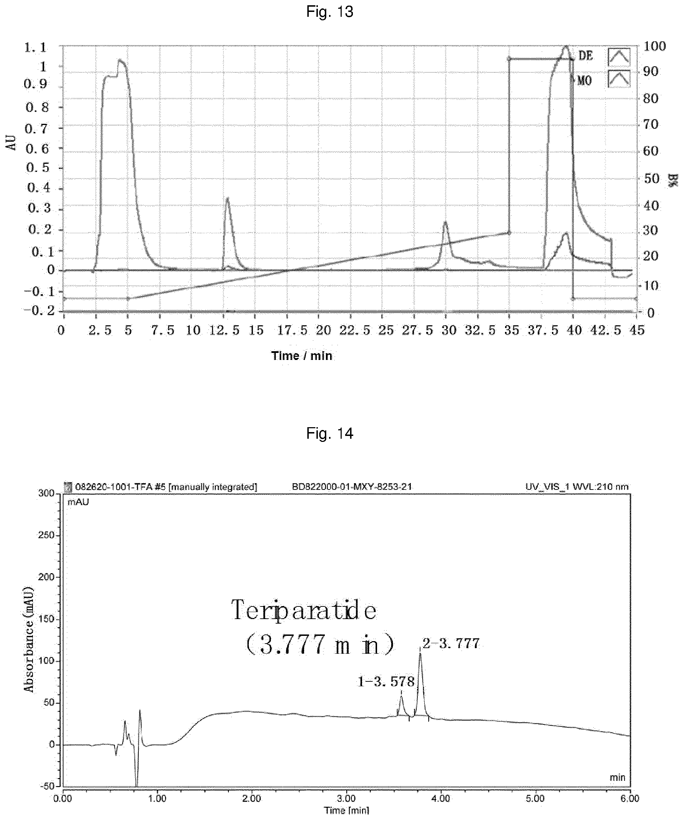

A gradient elution method used for Teri was as follows: 5 min with 5%B, 35 min with 30%B, 47.8 min with 42.8%B, 50.5 min with 42.8%B, 57.7 min with 50%B, 67.7 min with 95%B, 40 min with 95%B, ultraviolet detector of 210 nm, flow rate of 25 mL/min, temperature of 25°C. Results were shown in FIG. 13: Teri was eluted around 43 min. The sample was then collected, subjected to rotary evaporation to remove most of the solvent, and then freeze-dried. The purity was detected by HPLC, with an analytical column: Eclipse plus C18 4.6×100 mm 3.5 µm, 210 nm detection, flow=1.5 ml/min, elution gradients of 0 min with 10%B, 9 min with 95%B, 12 min with 100% B, 12.1 min with 10%B, 15 min with 10% B. Results showed that the purity of Teri reached -78% (FIG. 14).

A Liraglutide precursor was prepared by this method: 3-4 mg of a Liraglutide precursor with a purity of 98% could be obtained from 1 g of a shake flask fermentation bacterial sludge. By this method, 1.26 mg of Nesi with a purity of 98% could be prepared from 1 g of a bacterial sludge. By this method, 1.55 mg of Teri with a purity of 78% could be prepared from 1 g of a bacterial sludge. Further fermentation optimization to improve the expression level of fusion proteins is expected to further improve the yield of target polypeptides.

### Embodiment 11

### Mass spectrometry detection of molecular weight of polypeptide

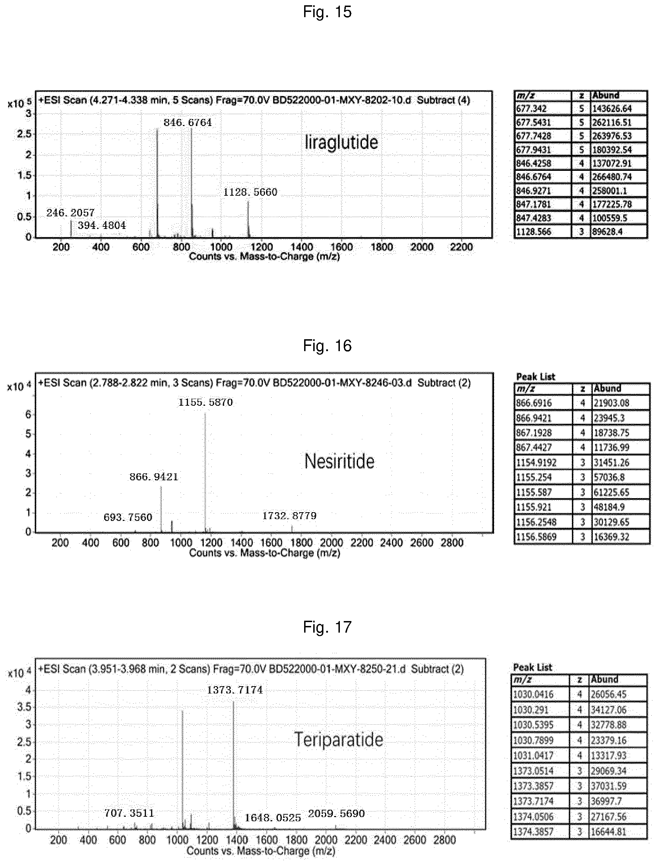

The molecular weights of the prepared polypeptides (products in Embodiment 10) were analyzed by LC-MS. The specific process was as follows: a sample was first separated by HPLC column: Agilent ZORBAX Edipse Plus C18, 4.6*100 mm, 3.5 µm, Mobile phase A: 0.1% trifluoroacetic acid, Mobile phase B: 0.1% trifluoroacetic acid-acetonitrile solution, a gradient elution method used: 0 min with 10%B, 9 min with 95%B, 12 min with 100%B, 12.1 min with 10%B, 15 min with 10%B, column temperature of 40°C, ultraviolet detector of 210 nm, flow rate of 1.5 mL/min. Components separated by HPLC were subjected to structural identification by Agilent 6200 series time-of-flight Liquid Chromatograph Mass Spectrometer. An electrospray ionization source (Dual AJS ESI) was used, with positive ion mode detection, a spray pressure of 35 psig, an ion source temperature of 300°C, a dry gas (N2) flow rate of 10 L/min, a scanning range of 100-2000 m/z, and a fragmentation voltage of 70 V, and mass spectrometry data was collected and processed by Analyst software. A theoretical molecular weight of Lira was 3383.7 Da, and a molecular weight analyzed by mass spectrometry was 3382.7 (FIG. 15). A theoretical molecular weight of Nesi was 3464.8, and a molecular weight analyzed by mass spectrometry was 3463.7 (FIG. 16). A theoretical molecular weight of Teri was 4117.7, and a molecular weight analyzed by mass spectrometry was 4118.7 (FIG. 17).

The above-mentioned descriptions are only preferred embodiments of the present invention and are not used to limit the present invention. For those skilled in the art, the present invention can have various modifications and changes. Any modification, equivalent replacement, improvement, etc., made within the spirit and principle of the present invention should be included in the scope of protection of the present invention.

## Claims

1. A preparation method for a polypeptide, wherein the preparation method comprises the following steps:
constructing an engineering strain for fusion-expressing a polypeptide gene with a Sumo tag, and inducing the engineering strain to soluble-express the polypeptide;
obtaining a fusion protein containing a polypeptide precursor from the engineering strain by purification;
cleaving the fusion protein containing the polypeptide precursor by using a Ulp1 protease to remove the Sumo tag; purifying a cleavage product of the Ulp1 protease by a method of acetonitrile combined with heating precipitation or a method of hexafluoroisopropanol precipitation, to obtain the polypeptide.

2. The preparation method according to claim 1, wherein the polypeptide is a Liraglutide precursor, a Nesiritide or a Teriparatide.

3. The preparation method according to claim 1, wherein the Ulp1 protease is obtained by constructing a Ulp1 protease expression strain and inducing expression, wherein the Ulp1 protease is co-expressed with a chaperone.

4. The preparation method according to claim 3, wherein the chaperone is a GroEUS chaperone.

5. The preparation method according to claim 1, wherein the method of acetonitrile combined with heating precipitation comprises: adjusting pH of the cleavage product of the Ulp1 protease to 5.6, then adding acetonitrile, after mixing uniformly, performing heat treatment at 60-80°C for 0.5-3 h, and then centrifuging to separate supernatant and precipitate.

6. The preparation method according to claim 5, wherein the acetonitrile is acetonitrile aqueous solution of which a mass percentage content is 20-70%.

7. The preparation method according to claim 1, wherein the step of obtaining the fusion protein containing the polypeptide precursor from the engineering strain comprises: obtaining a crude solution after ultrasonic disruption, centrifugation, and filtration with membrane of the engineering strain, and then purifying with an affinity chromatography or an anion column to obtain the fusion protein.

8. The preparation method according to claim 1, wherein the method of hexafluoroisopropanol precipitation comprises: adjusting pH of the cleavage product of the Ulp1 protease to 5.6, then adding hexafluoroisopropanol, after mixing uniformly, precipitating at a room temperature for 1 h, and then centrifuging to separate supernatant and precipitate.

9. The preparation method according to claim 8, wherein the hexafluoroisopropanol is hexafluoroisopropanol aqueous solution of which a mass percentage content is 20-70%.

10. The preparation method according to claim 9, wherein the hexafluoroisopropanol is the hexafluoroisopropanol aqueous solution of which the mass percentage content is 50%.

11. The preparation method according to claim 1, wherein the preparation method further comprises a step of purifying a target polypeptide by a high performance liquid chromatography (HPLC).
